# EUROPEAN PATENT APPLICATION

(11) **EP 4 424 358 A1**
(43) Date of publication of application: **04.09.2024**
(21) Application number: 23190337.8
(22) Date of filing: 08.08.2023
(51) Int. Cl.: A61N 1/05, A61N 1/36, A61N 1/378

(54) **NEUROSTIMULATOR TO AID IN RECOVERY FROM STROKE OR OTHER LIMITATIONS IN HUMAN FUNCTION**

(30) Priority: 03.03.2023 US 202318117299
(71) Applicant: Cortigent, Inc., Valencia, CA 91355 (US)
(72) Inventor: DATTA, Proyag, Thousand Oaks (US); OK, Jerry, Canyon Country (US); PATEL, Uday, Los Angeles (US)
(74) Representative: Mewburn Ellis LLP

(57) **Abstract**

Briefly, and in general terms, the present invention is directed to a cortical stimulator for neuromodulation. The cortical stimulator is configured so as to stimulate or block the operation of a portion of the cortex, for example, but not limited to, the motor cortex of a subject who has suffered a stroke. The present disclosure of a cortical stimulator includes a flexible circuit electrode array connected to an electronics package and a coil inductively coupled to an external coil for power and data. The present invention includes an electrode array significantly larger than the area intended to be stimulated. The larger electrode array allows a clinician to select the location of stimulation after completion of the surgery.

## Description

### FIELD OF THE INVENTION

The present disclosure relates to cortical neural stimulation. More particularly, it relates to an apparatus, method of use, and method of manufacture for a system including an implantable electrode array, connected electronics package, implantable coil, and associated external coil and support electronics for a cortical stimulator to aid in stroke recovery, and a variety of other conditions.

### BACKGROUND OF THE INVENTION

Approximately 7.6 million Americans over the age of 20 have reported a history of stroke. While rehabilitation therapy may restore some function for many stroke victims, approximately 40% of these patients suffer motor impairments require special care. Consequently, stroke is a leading cause of long-term disability, costing the American economy $38 billion per year due to under-employment and $35 billion in direct healthcare costs. Each year, 610,000 Americans have a first stroke and the age of first stroke is trending younger. This trend portends a growing burden due to under-employment, estimated to reach $56.5 billion per year by 2030.

Rehabilitation therapy after stroke attempts to restore the functions lost due to the patient's stroke. These functions may include movement, speech, strength, and activities of daily living. Conventional rehabilitation therapy for upper extremity function typically utilizes a variety of strategies including strength, balance, stretching, and manual dexterity exercises, and functional task practice of activities of daily living (ADLs) using either the impaired or the less-affected side. Rehabilitation therapy typically follows a course of in-patient rehabilitation followed by out-patient rehabilitation either at a clinic or at home. While most functional gains occur during the acute phase post-stroke, evidence suggests that some degree of functional benefit from therapy can occur beyond 12 months post-stroke.

To enhance the efficacy of stroke rehabilitation for upper extremity function, several technological interventions have been evaluated in clinical trials. Repetitive transcranial magnetic stimulation combined with rehabilitation was explored in the Navigated Inhibitory rTMS to Contralesional Hemisphere (NICHE) trial. Electromyographytriggered neuromuscular stimulation was explored in the Explaining PLastICITy after stroke trial. The effect of virtual reality and video games on upper limb motor capacity during the subacute stage of stroke were evaluated in Effectiveness of Virtual Reality Exercises in STroke Rehabilitation (EVREST). Virtual Reality Training for Upper Extremity in Subacute Stroke (VIRTUES) and a trial by Adie and colleagues. Tele-rehabilitation during the subacute and chronic stages of stroke was investigated by Cramer and colleagues. The Robot Assisted Training for the Upper Limb after Stroke (RATULS) trial investigated the effects of robot-assisted therapy on upper limb motor capacity with participants primarily at the chronic stage. The execution of these trials illustrate the feasibility of integrating technological interventions into rehabilitation therapy; however, all of these trials reported similar benefits to those produced by a matched dose of conventional therapy, recreational activities, or sham stimulation.

Vagus nerve stimulation (VNS) combined with rehabilitation therapy has been shown to improve functional outcomes. VNS activates multiple global neuromodulatory systems throughout the brain. For post-stroke rehabilitation, cholinergic and monoaminergic modulation of motor cortex are hypothesized to enhance the reorganization potential following stroke. Pairing VNS stimulation with rehabilitation (VNS-REHAB) has been shown to elicit a small but clinically meaningful improvement in motor function compared to sham-stimulation controls. This approach recently received FDA approval and is currently available in the market. Still, not all patients respond to VNS-REHAB; 47% achieved a clinically meaningful response versus 24% in controls (rehabilitation therapy only). This leaves a significant number of stroke survivors with a persistent therapeutic need.

Electrical stimulation of the primary motor cortex offers the possibility of much greater target specificity than vagus nerve stimulation. Prior clinical trials combining electrical stimulation of the motor cortex with rehabilitation have been completed with a single channel of stimulation with an effective activation area of 1.8 cm². With continuous stimulation during rehabilitation sessions, this technology demonstrated significant improvements over conventional therapy alone in Phase I and II trials. However, the Everest Phase III trial was unsuccessful in demonstrating efficacy. Combined, these trials established the safety and feasibility of combining cortical stimulation with rehabilitation. The failure of efficacy in Phase III has been attributed, at least in part, to incorrect localization of the stimulating array.

### SUMMARY OF THE INVENTION

Briefly, and in general terms, the present invention is directed to a cortical stimulator for neuromodulation. The cortical stimulator is configured so as to stimulate or block the operation of a portion of the cortex, for example, but not limited to, the motor cortex of a subject who has suffered a stroke.

A neurostimulator to support stroke rehabilitation, the neurostimulator comprising: an external portion including: a processor adapted to generate stimulation patterns and select drivers for those stimulation patterns, the processor driving an external coil, the external coil configured to provide power and data to an implantable coil, the data including the stimulation patterns and selected drivers; an implantable portion including: the implantable coil configured to receive the power and data from the external coil and to provide the power and data to an electronic circuit within a hermetic package, wherein the electronic circuit includes a plurality of drivers, each driver electrically connected to a respective subset of a plurality of electrodes; and an electrode array adapted to cover a simulation region of a motor cortex of a brain, the electrode array including the plurality of electrodes, each electrode covering a respective portion of the stimulation region of the motor cortex of the brain.

The present disclosure of a cortical stimulator may include a flexible circuit electrode array connected to an electronics package and a coil inductively coupled to an external coil for power and data. The present invention may include an electrode array which is larger (optionally, significantly larger) than the area intended to be stimulated. The larger electrode array allows a clinician to select the location of stimulation after completion of the surgery. It should be noted that a "subset" of electrodes as used throughout this patent application may refer to a single electrode, or a plurality of electrodes.

The data provided by the processor and external coil to the implantable portion includes the selected drivers. The manner in which these drivers are selected is set out later in this patent application. Each of the drivers in the electronic circuit may be configured to activate the subset of electrodes to which it is electrically connected. Specifically, each driver may be activated, wherein activation of a driver causes it to activate the subset of electrodes to which it is connected. The electronic circuit may comprise a controller which is configured to determine which drivers to activate, based on the received data. For example, each driver may have a driver identifier, and the data may comprise the driver identifiers of the drivers to be activated. In response to receiving the data, the controller may be configured to activate the drivers corresponding to the driver identifiers in the data. In some cases, the controller may comprise the drivers.

The neurostimulator of the first aspect of the invention is to support stroke rehabilitation. Stroke patients often have reduced motor function, which may lead to difficulty in performing simple tasks such as grasping an object. Neurostimulators according to the present invention can be used to assist stroke patients by stimulating regions of the motor cortex which give rise to muscle contractions in selected muscles in, for example, the upper extremity musculature, thereby enabling them to perform tasks with which they were previously having difficulty. Each portion of the stimulation region of the motor cortex of the brain may correspond to a muscle, for example a muscle in the upper extremity musculature, which is activated in response to a suprathreshold electrical stimulation of that portion. Herein, a "suprathreshold electrical stimulation" refers to a stimulation which has a sufficiently high magnitude to cause firing of an action potential of neurons within that portion of the stimulation region of the motor cortex.

In order to determine which drivers to select (and consequently, which portions of the stimulation region to stimulate), the processor may be configured to consult a somatotopic map which relates the subsets of electrodes to the respective muscles (or sets of muscles) which are activated by application of a suprathreshold electrical stimulation to the portion of the stimulation region covered by those electrodes. The somatotopic map may be in the form of a lookup table (or equivalent) stored in a memory in the external portion of the neurostimulator. For example, the lookup table may store first data, second data, and a plurality of associations relating subsets of the first data to subsets of the second data. The first data may comprise data identifying the drivers and/or the subsets of electrodes. The second data may comprise data identifying the muscles and/or the portions of the stimulation region of the motor cortex. More specifically, each item of the first data may corresponding to a respective item of the second data, thereby indicating the correspondence between the drivers/subsets of electrodes and the muscles/portions of the stimulation region.

The processor may be configured to determine which drivers to select in response to a received input. For example, the received input may comprise an action to be performed by the user, for example a body movement. In such cases the processor may be configured to determine which drives to select in order to achieve the body movement in question, i.e. the drivers which are associated with the muscles, activation of which is required to achieve the body movement in question. As an example, a user may select bend arm and the processor will select the neurons associated with the bicep, or the user may select straighten arm and the processor will select the neurons associated with the triceps. The processor may be configured to provide a more general tonic stimulation to a provide stimulus for regrowth of neural connections, or the processor may be configured to provide a specific input to accomplish a task.

We now discuss the manner in which the somatotropic map may be generated. In response to a suprathreshold electrical stimulation in a corresponding region of the motor cortex, a muscle elsewhere in the body may be activated, i.e. a muscular response may be elicited in the muscle. This may mean that the muscle contracts. This may lead to the muscle twitching or jerking. In addition to e.g. a visual twitch of the muscle, a motor evoked potential may be elicited in the muscle. Herein, the term "motor evoked potential" refers to an action potential which is elicited within a muscle in response to the suprathreshold electrical stimulation. These motor evoked potentials are measurable using standard techniques.

A second aspect of the invention may provide a method of generating a somatotopic map using the neurostimulator of the first aspect of the invention, the method comprising: (a) using the processor, selecting a driver of the plurality of drivers and a test stimulation pattern; (b) using the external coil, providing power and data including the selected driver of the plurality of drivers and the test stimulation pattern to the implantable portion, the implantable portion having been implanted such that the electrode array covers the stimulation region of the motor cortex, and each subset of electrodes covers a respective portion of the stimulation region; (c) using the driver, activating the subset of electrodes to which the driver is connected, thereby applying a suprathreshold electrical stimulation to the respective portion of the stimulation region covered by that subset of electrodes; (d) detecting a muscular response elicited by the application of the suprathreshold electrical stimulation; I using the processor, recording an association between the driver and the muscular response; (f) using the processor, selecting another driver and repeating steps (a) to I until all drivers have been selected.

Optionally, the method may further comprise storing a somatotopic map comprising all of the associations between the drivers and the muscular responses, for example in a memory of the external portion of the neurostimulator. Detecting the muscular response may comprise detecting or measuring a motor evoked potential in the musculature or recording visual muscle twitch feedback.

Returning to optional features of the first aspect of the invention, the somatotopic map may have been generating using a method according to the second aspect of the invention.

It is desirable that the stimulation region covers as much of the motor cortex as possible. Therefore, the electrode array may be larger than an accessible area of the motor cortex, to ensure that the electrode array covers all of the accessible region of the motor cortex. In the context of treating stroke patients, implementations of the first aspect of the invention are particularly effective when applied to enabling or strengthening hand movements which may be required for e.g. picking up objects, writing, eating, drinking and the like. In view of this, the electrode array may be configured to cover the upper extremity region of the motor cortex, i.e. the region of the motor cortex which is responsible for controlling (e.g. activating) muscles in the upper extremities.

We now provide more details regarding the structure of the electrode array.

The implantable portion may comprise a flexible circuit which comprises the electrode array at a first end. The flexible circuit may comprise a bond pad region at a second end. The bond pad region may comprise one or more bond pads. The flexible circuit may further comprise a cable connecting the electrode array to the bond pad region.

The flexible circuit may comprise electrical traces inside a polymer body. For example, the flexible circuit may comprise electrical traces sandwiched between two layers (i.e. a top layer and a bottom layer) of polymer. The polymer may be polyimide. The electrical traces may comprise the plurality of electrodes and may comprise the one or more bond pads. The electrical traces may be metal traces, such as platinum traces. One or more of the top layer and the bottom layer may comprise openings exposing the plurality of electrodes and/or the one or more bond pads.

The pressure applied against cortical neurons, or other neural tissue, by an electrode array is critical. Too little pressure causes increased electrical resistance between the array and cortex, along with electric field dispersion. Too much pressure may block blood and/or cerebral fluid flow causing cortical ischemia and hemorrhage, possibly resulting in an additional stroke. Common flexible circuit fabrication techniques such as photolithography generally require that a flexible circuit electrode array be made flat. Since the cortex includes multiple approximately spherical lobes, a flat array will necessarily apply more pressure near its edges, than at its center. Further, the edges of a flexible circuit polymer array may be quite sharp and cut the delicate cortical tissue. In order to combat this, the electrode array may be pre-curved (i.e. formed to have a curved shape). More specifically, the electrode array may be pre-curved to conform to the surface of the neural tissue which it is meant to cover, e.g. the stimulation region of the motor cortex. Here, "conform" should be understood to mean that the curvature of the electrode array matches or substantially matches the curvature of the surface in question. A combination of the pre-curvature and the flexible nature of the electrode array ensures that the electrodes are in close contact with the neural tissue. By pre-curving the electrode array, though, the stresses in the electrode array are reduced as it flexes to conform to the neural tissue, reducing the likelihood that the traces break. The forces exerted on the neural tissue by the edges of the electrode array are also reduced as a result of the pre-curvature.

In order to further reduce the forces exerted on the neural tissue by the edges of the electrode array, it may be coated with a more flexible or compliant material, such as a more flexible polymer. Specifically, either the edges, or a portion of the edges may be covered with a more flexible or more compliant material. Additionally or alternatively, the entire electrode array may be coated with such a material, the coating comprising openings to expose the plurality of electrodes and/or the one or more bond pads. The material may be polydimethylsiloxane (PDMS), or silicone.

The first and second aspects of the invention are related to the use of a neurostimulator for rehabilitation after a stroke. It will be acknowledged, however, that neurostimulators according to the present invention may be used for purposes other than supporting stroke rehabilitation. The third aspect of the invention provides a more general neurostimulator.

Specifically, a third aspect of the present invention provides a neurostimulator comprising: an external portion including: a processor adapted to generate stimulation patterns and select drivers for those stimulation patterns, the processor driving an external coil, the external coil configured to provide power and data to an implantable coil, the data including the stimulation patterns and selected drivers; and an implantable portion including: the implantable coil configured to receive the power and data from the external coil and to provide power and data to an electronic circuit within a hermetic package, wherein the electronic circuit includes a plurality of drivers, each driver electrically connected to a respective subset of a a plurality of electrodes; and an electrode array adapted to cover a stimulation region of a cortex of a brain, the electrode array including the plurality of electrodes, each electrode covering a respective portion of the stimulation region of the cortex of the brain.

The optional features set out above in respect of the first aspect of the invention or the second aspect of the invention also apply to the third aspect of the invention, except where clearly technically incompatible, or where context dictates otherwise.

Neurostimulators according to the third aspect of the invention may be used for many functions. Accordingly, the electrode array may be configured to cover various different regions of the cortex. For example, the electrode array may be configured or adapted to cover the prefrontal cortex (e.g. for treatment of depression or addiction), the auditory cortex (e.g. for treatment of tinnitus or deafness), the eloquent cortex (e.g. for treatment of epilepsy), the speech cortex (also known as Broca's area, e.g. for treatment of aphasia), or any other suitable region of the cortex. Stimulation of the motor cortex may also be useful for the treatment of traumatic brain injury.

Other features and advantages of the invention will become apparent from the following detailed description, taken in conjunction with the accompanying drawings, which illustrate, by way of example, the features of the invention.

### BRIEF DESCRIPTION OF DRAWINGS

The accompanying drawings, which are incorporated into and constitute a part of this specification, illustrate one or more embodiments of the present disclosure and, together with the description of example embodiments, serve to explain the principles and implementations of the disclosure.
FIG. 1 is a bottom view of the stroke rehabilitation implant.
FIG. 2 is a top perspective view of the stroke rehabilitation implant.
FIG. 3 is a side view of the stroke rehabilitation implant.
FIG. 4 is a view of the stroke rehabilitation implant as implanted on the motor cortex of the brain.
FIG. 5 is a view of an example human brain, including area that can be stimulated to support multiple therapeutic actions.

### DETAILED DESCRIPTION

While general regions of somatotopic representation (i.e. head, arm, leg) in motor cortex as depicted in the homunculus are well accepted, evidence suggests that the somatotopic representation of distinct body segments may be distributed broadly and have multiple representations within the motor cortex. By implanting an array of electrodes over the upper extremity region of the motor cortex, one will be able to map the somatotopic arrangement in unprecedented detail in a controlled, methodical, and reproducible outpatient setting. By recording motor evoked potentials from upper extremity musculature while stimulating individual electrodes or groups of adjacent electrodes, one will be able to generate a motor map that identifies which muscles are activated by suprathreshold stimulation of each electrode or group of electrodes. These motor evoked potentials can serve as a biomarker of physiological response to combined stimulation and rehabilitation. Adjacent electrodes may have functionally distinct motor potentials and may be utilized distinctly during different tasks. The individualized motor maps can be used to determine appropriate stimulation targets for specific tasks. During rehabilitation, these tasks can be practiced while the corresponding stimulation is provided at subthreshold levels. The electrode array can be placed either epidurally or subdurally over the target region of the cortex. To maximize specificity of stimulation, the array should be implanted subdurally.

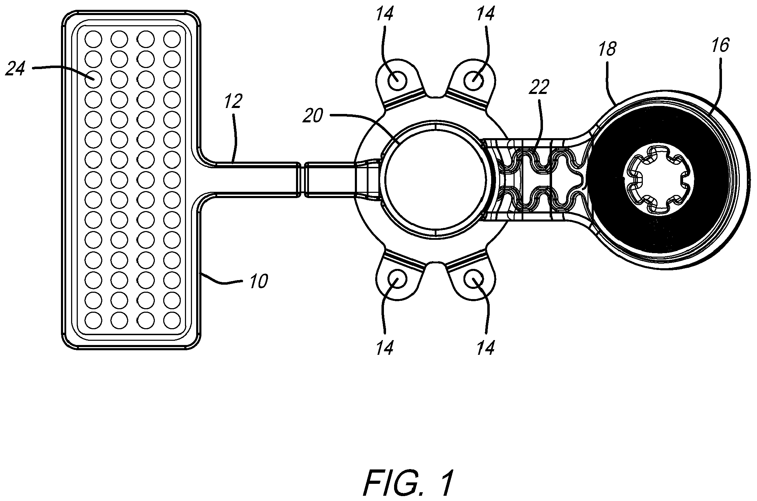

Turning to the drawings **Figure 1** shows a bottom view (the side facing the brain) of the cortical stimulator. Power and data is transferred to the implanted cortical stimulator through an inductive coil **16.** The inductive coil is electrically connected in an implantable pulse generator (IPG) within a hermetic package **20** through leads **22.** The coil and leads are over molded with a polymer **18,** preferably silicone. The hermitic package **20** is adapted to be attached to a skull through voids **14** adapted to accept skull screws (not shown). A flexible circuit electrode array **10** is attached electrically connected to the IPG in the hermetic package **20** by a cable **12.** Each electrode **24** has an independent trace leading through the electrode array **10** and cable **12** back to the IPG. It should be noted that the electrode array **10** and cable **12** are formed from a single flexible circuit.

The flexible circuit is preferably a sandwiched polymer body with the electrode array **10** at one end bond pads for connecting the array to the electronics package **20** at the other end, and traces through a cable **12** connecting bond pads to electrodes **24.** Polymer materials are useful as electrode array bodies for neural stimulation. They are particularly useful for cortical stimulation for many purposes. Regardless of which polymer is used, the basic construction method is the same. A layer of polymer is laid down, commonly by some form of chemical vapor deposition, spinning, meniscus coating or casting. A layer of metal, preferably platinum, is applied to the polymer and patterned to create electrodes bond pads and leads connecting electrodes to bond pads. Patterning is commonly done by photolithographic methods. A second layer of polymer is applied over the metal layer and patterned to leave openings for the electrodes **24** and bond pads, or openings are created later by means such as laser ablation. Hence the array **10,** its supply cable **12** and bond pads are formed of a single body. Multiple alternating layers of metal and polymer may be applied to obtain more metal traces within a given width.

The pressure applied against cortical neurons, or other neural tissue, by an electrode array is critical. Too little pressure causes increased electrical resistance between the array and cortex, along with electric field dispersion. Too much pressure may block blood flow causing cortical ischemia and hemorrhage. Common flexible circuit fabrication techniques such as photolithography generally require that a flexible circuit electrode array be made flat. Since the cortex is approximately spherical, a flat array will necessarily apply more pressure near its edges, than at its center. Further, the edges of a flexible circuit polymer array may be quite sharp and cut the delicate cortical tissue. With most polymers, it is possible to curve them when heated in a mold. By applying the right amount of heat to a completed array, a curve can be induced that matches the curve of the cortex. With a thermoplastic polymer such as liquid crystal polymer, it may be further advantageous to repeatedly heat the flexible circuit in multiple molds, each with a decreasing radius. Further, it is advantageous to add material along the edges of a flexible circuit array. Particularly, it is advantageous to add material that is more compliant than the polymer used for the flexible circuit. While described as a flexible circuit electrode array, more conventional techniques such as silicone covered wires can also be used.

It is further advantageous to make a thinner polyimide array as the core layer and coat the entire array with a thin layer of Polydimethylsiloxane (PDMS) and to open up the electrode sites and for plating the electrodes with Pt gray or other metal electrode materials. Importantly, a flexible circuit is sufficiently flexible to follow the shape of the cortex.

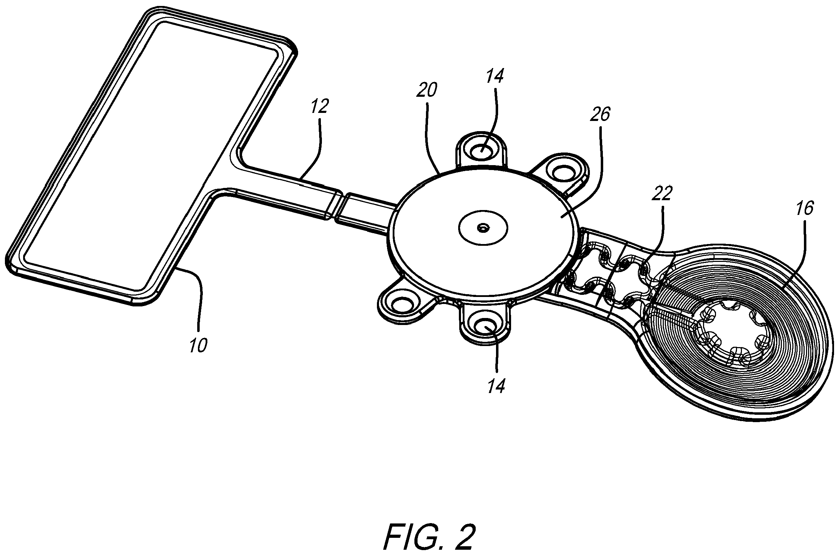

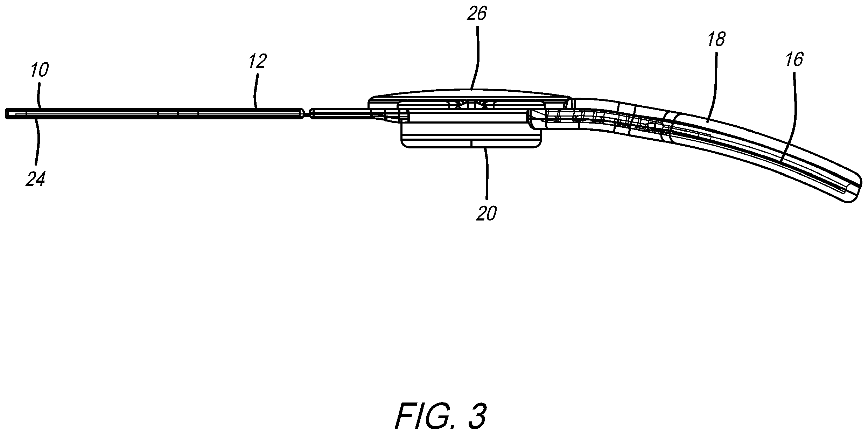

**Figure 2** shows a top perspective view of the implantable cortical stimulator, and **Figure 3** shows a side view of the implantable cortical stimulator. The hermetic package **20** includes a domed lid **24.** The domed lid **26** increases impact resistance and reduced irritation of the scalp when the implantable cortical stimulator is implanted. The cortical implant is described in more detail in relation to applicants' cortical visual prosthesis described in US patents 9,861,820 and 9,949,376, which are incorporated herein by reference.

The present invention includes an improved hermetic electronics package for implantation in the human body. A cover is bonded to a substrate having electronic connection vias (manufactured as a "via substrate" using co-fired ceramic technology) such that the cover and via substrate form a hermetic package. In the assembly of hybrid circuits for biomedical devices, surface mount components are often used. The present disclosure includes thin chip components in place of traditional surface mount discrete components. The thin chip components may include stacked capacitors having a low profile, for example, a total height between twenty-five and one hundred-fifty micrometers. The stacked capacitors may be high density trench capacitors and/or metal-on-semiconductor capacitors positioned on an integrated circuit chip. The thin chip components may be a metal-insulator-metal capacitor, wherein the metal-insulator-metal capacitor has a tunable capacitance value and/or is a binary capacitor array. Due to the fact that many biomedical devices are implanted in biological tissues, the use of components with a lower height and reduced lateral footprint Is advantageous. Additionally, when using surface mount capacitors to provide tuning capacitance values, it is necessary to prescreen capacitors to be selected for assembly in-process rather than at kitting. The present disclosure includes the advantageous use of components that are tunable in-situ.

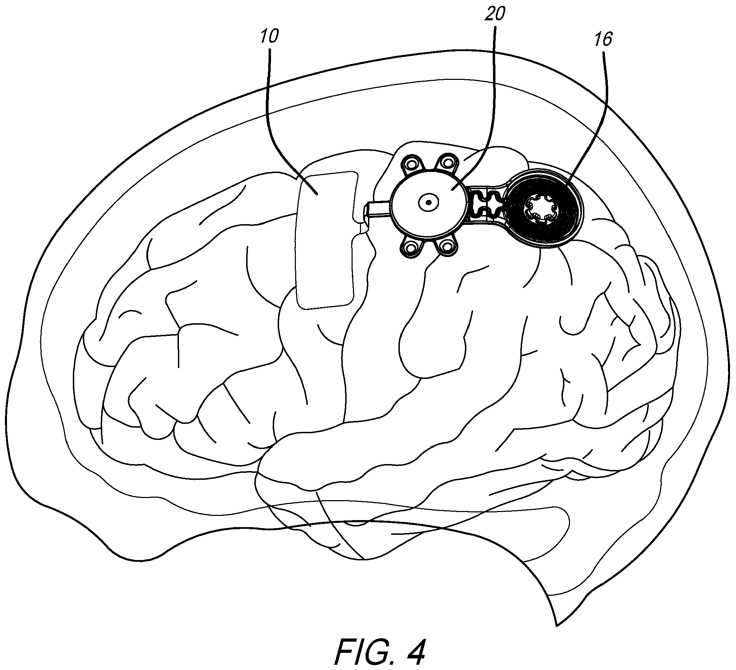

**Figure 4** shows the implantable cortical stimulator and how it is intended to be implanted. The cable **12** pierces the skull and dura to allow the electrode array to be in direct contact with the brain. The hermetic package is placed within an inset burr hole in the skull so it is near flush with the skull. The coil **16** lies on the surface of the skull or within a shallow hollowed out portion. All components, electrode array **10,** cable **12** hermetic package **20** and coil **16,** remain under the scalp to avoid infection. The most important aspect of successful cortical stimulation for stroke rehabilitation is precise location of stimulation. The present invention includes an electrode array **10** that covers an area slightly larger than the motor cortex, 60 independent electrodes **24,** and a 60 channel IPG. This allows a clinician to determine the best location for stimulation in the clinic after surgery is completed.

Therapy may only require one or two channels of active stimulation of a small region of cortex near the array. Herein, the term "channel" may be understood to refer to an electrode linked to a driver. The system may comprise multiplexers, in order to enable the presence of more independently controllable electrodes than drivers. The advantage of the 60 channel stimulator of the present invention is to allow a clinician to adjust the location of stimulation post surgery, or change the location of stimulation if the initial indication proves incorrect, or provide differing therapy as recovery progresses. The location of stimulation can be adjusted by selecting either a single electrode or groups of electrodes and by using monopolar, bipolar or multipolar configurations. Sequential stimulation of multiple electrodes can be used to target the execution of more complicated movements. It has been established through clinical study that a tonic stimulation over a large area of the motor cortex in conjunction with rehabilitation promotes recovery of function. Such stimulation can be provided by the present invention. The system of the present invention can also provide further therapeutic benefit by targeting stimulation to a desired movement. As an example, in a patient who has having difficulty grasping an object, a clinician can target stimulation to the specific area of the cortex commanding a contraction of muscles responsible for grasping. Repetition of applying the stimulation and the patient attempting to grasp may result in retraining of the neural circuits to improve the patient's ability to grasp independently.

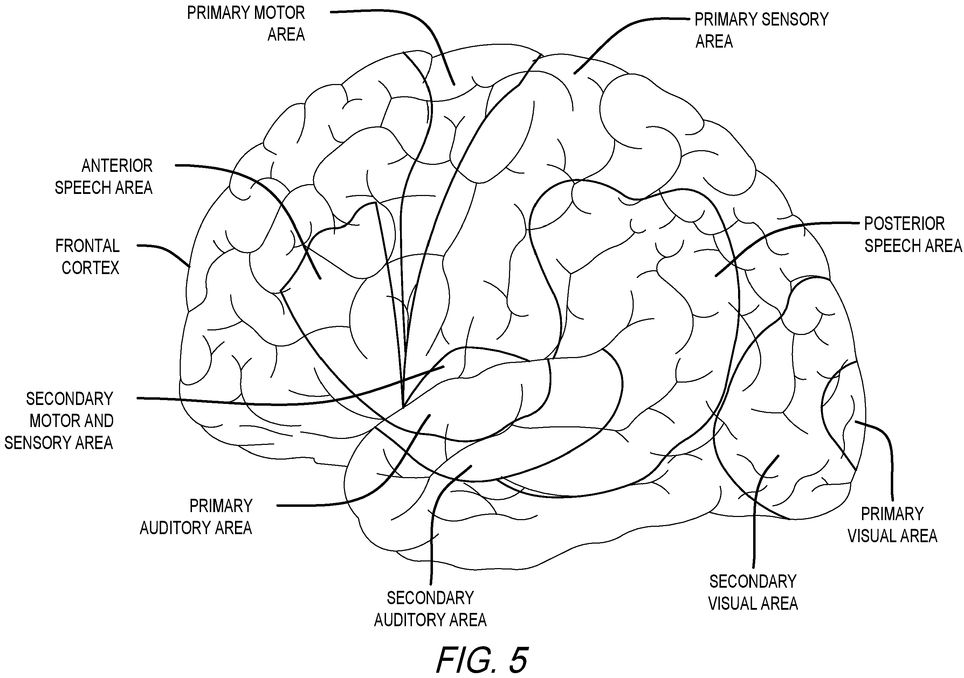

While described as a cortical stimulator for stroke recovery, the present invention is a modification of applicant's cortical stimulator for artificial vision. Referring to **Figure 5****,** the present invention can be further modified to provide benefit for treating other conditions by modifying the electrode array to cover different portions of the cortex, and providing different control software to provide different stimulation patterns. Examples include stimulating the auditory cortex for tinnitus or deafness, covering the prefrontal cortex for depression or addiction, covering Broca's area for aphasia, covering the eloquent cortex for epilepsy, or covering the motor cortex for traumatic brain injury. In each case, the 60 channel stimulator of the present invention allows for covering the entire target area and fine tuning the location of stimulation by clinical experimentation post surgery.

A number of embodiments of the disclosure have been described. Nevertheless, it will be understood that various modifications may be made without departing from the spirit and scope of the present disclosure. Accordingly, other embodiments are within the scope of the following claims.

The examples set forth above are provided to those of ordinary skill in the art as a complete disclosure and description of how to make and use the embodiments of the disclosure, and are not intended to limit the scope of what the inventor/inventors regard as their disclosure.

Modifications of the above-described modes for carrying out the methods and systems herein disclosed that are obvious to persons of skill in the art are intended to be within the scope of the following claims. It is to be understood that the disclosure is not limited to particular methods or systems, which can, of course, vary. It is also to be understood that the terminology used herein is for the purpose of describing particular embodiments only, and is not intended to be limiting. As used in this specification and the appended claims, the singular forms "a," "an," and "the" include plural referents unless the content clearly dictates otherwise. The term "plurality" includes two or more referents unless the content clearly dictates otherwise. Unless defined otherwise, all technical and scientific terms used herein have the same meaning as commonly understood by one of ordinary skill in the art to which the disclosure pertains.

## Claims

1. A neurostimulator to support stroke rehabilitation, the neurostimulator comprising:
an external portion including:
a processor adapted to generate stimulation patterns and select drivers for those stimulation patterns, the processor driving an external coil, the external coil configured to provide power and data to an implantable coil, the data including the stimulation patterns and selected drivers;
an implantable portion including:
the implantable coil configured to receive the power and data from the external coil and to provide the power and data to an electronic circuit within a hermetic package, wherein the electronic circuit includes a plurality of drivers, each driver electrically connected to a respective subset of a plurality of electrodes; and
an electrode array adapted to cover a simulation region of a motor cortex of a brain, the electrode array including the plurality of electrodes, each electrode covering a respective portion of the stimulation region of the motor cortex of the brain.

2. The neurostimulator according to claim 1, wherein:
each of the driver in the electronic is configured to activate the subset of electrodes to which it is electrically connected; and
activation of a driver is configured to cause the driver to active the subset of electrodes to which it is connected.

3. The neurostimulator according to claim 2, wherein:
the electronic circuit further comprises a controller, the controller configured to determine which drivers to activate based on the received data comprising the selected drivers.

4. The neurostimulator according to any one of claims 1 to 3, wherein:
the processor is configured to select drivers by consulting a somatotopic map, the somatotopic map relating the subsets of electrodes of the electrode array to the respective muscles or sets of muscles which are activated in response to the application of a suprathreshold electrical stimulation to the portion of the stimulation region covered by that subset of electrodes.

5. The neurostimulator according to claim 4, wherein:
the processor is configured to select the drivers in response to a received user input.

6. The neurostimulator according to any one of claims 1 to 5, wherein the driver selection is based on visual observation of muscle twitch feedback or measurement of motor evoked potentials.

7. The neurostimulator according to any one of claims 1 to 6, wherein the electrode array is configured to cover the upper extremity region of the motor cortex.

8. The neurostimulator according to any one of claims 1 to 7, wherein the electrode array is larger than an accessible area of the motor cortex to ensure full coverage of the motor cortex.

9. A neurostimulator comprising:
an external portion including:
a processor adapted to generate stimulation patterns and select drivers for those stimulation patterns, the processor driving an external coil, the external coil configured to provide power and data to an implantable coil, the data including the stimulation patterns and selected drivers; and
an implantable portion including:
the implantable coil configured to receive the power and data from the external coil and to provide power and data to an electronic circuit within a hermetic package, wherein the electronic circuit includes a plurality of drivers, each driver electrically connected to a respective subset of a a plurality of electrodes; and
an electrode array adapted to cover a stimulation region of a cortex of a brain, the electrode array including the plurality of electrodes, each electrode covering a respective portion of the stimulation region of the cortex of the brain.

10. The neurostimulator according to claim 9, wherein the electrode array is adapted to cover the prefrontal cortex, the auditory cortex, the eloquent cortex, or the speech cortex.

11. The neurostimulator according to any one of claims 1 to 10, wherein the implantable portion comprises a flexible circuit comprising the electrode array.

12. The neurostimulator according to claim 13, wherein the flexible circuit is a sandwich of polyimide layers with platinum traces, and over molded with polydimethylsiloxane.

13. A method of generating a somatotopic map using the neurostimulator of any one of claims 1 to 8, 11 and 12, the method comprising:
(a) using the processor, selecting a driver of the plurality of drivers and a test stimulation pattern;
(b) using the external coil, providing power and data including the selected driver of the plurality of drivers and the test stimulation pattern to the implantable portion, the implantable portion having been implanted such that the electrode array covers the stimulation region of the motor cortex, and each subset of electrodes covers a respective portion of the stimulation region;
(c) using the driver, activating the subset of electrodes to which the driver is connected, thereby applying a suprathreshold electrical stimulation to the respective portion of the stimulation region covered by that subset of electrodes;
(d) detecting a muscular response elicited by the application of the suprathreshold electrical stimulation;
(e) using the processor, recording an association between the driver and the muscular response;
(f) using the processor, selecting another driver and repeating steps (a) to (e) until all drivers have been selected.

14. The method of claim 13, wherein:
detecting the muscular response comprises detecting or measuring a motor evoked potential or recording visual muscle twitch feedback.

15. The method of claim 13 or claim 14, further comprising:
storing a somatotopic map comprising all of the associations between the drivers and the muscular responses in a memory of the external portion of the neurostimulator.
